# FASCICULE DE BREVET EUROPEEN

(11) **EP 3 057 929 B1**
(45) Date de publication et mention de la délivrance du brevet: **15.04.2020**
(21) Numéro de dépôt: 14796809.3
(22) Date de dépôt: 13.10.2014
(51) Int. Cl.: C07C 17/25, C07C 17/20, C07C 17/21, C07C 19/08, C07C 21/18

(54) **PROCÉDÉ DE PRODUCTION DE COMPOSES FLUORES**
VERFAHREN ZUR HERSTELLUNG FLUORIERTER VERBINDUNGEN
METHOD FOR PRODUCING FLUORINATED COMPOUNDS

(30) Priorité: 17.10.2013 FR 1360101
(43) Date de publication de la demande: 24.08.2016
(73) Titulaire: Arkema France, 92700 Colombes (FR)
(72) Inventeur: BONNET, Philippe, F-69007 Lyon (FR); COLLIER, Bertrand, F-69230 Saint-Genis-Laval (FR); DEUR-BERT, Dominique, F-69390 Charly (FR); GARRAIT, Dominique, F-69390 Charly (FR); PIGAMO, Anne, F-69340 Francheville (FR); WENDLINGER, Laurent, F-69510 Soucieu en Jarrest (FR)
(74) Mandataire: Leca, François Michel
(86) Numéro de dépôt international: PCT/FR2014/052593
(87) Numéro de publication internationale: WO 2015/055927

(56) Documents cités:
- WO-A1-2013/088195
- WO-A1-2014/159975
- CN-A- 101 074 185
- THAKUR ET AL: "Static Mixers in the Process Industries-A Review", CHEMICAL ENGINEERING RESEARCH AND DESIGN, ELSEVIER, AMSTERDAM, NL, vol. 81, no. 7, 1 August 2003 (2003-08-01) , pages 787-826, XP022536538, ISSN: 0263-8762, DOI: 10.1205/026387603322302968

## Description

### DOMAINE DE LA PRESENTE DIVULGATION

La présente divulgation concerne un procédé de production de composés fluorés, tels que des hydrofluorooléfines ou des hydrofluorocarbures par exemple, et une installation adaptée à la mise en œuvre de ce procédé.

### ARRIERE-PLAN TECHNIQUE

Il est connu de produire des hydrofluorooléfines ou des hydrofluorocarbures par fluoration d'hydrochlorooléfines ou d'hydrochlorocarbures notamment. Cette fluoration est généralement une fluoration catalytique utilisant l'acide fluorhydrique comme agent fluorant.

La réaction de fluoration doit généralement être effectuée à une température élevée (plus de 300°C) en phase gazeuse. Par conséquent, il est connu de chauffer, de vaporiser et de surchauffer les réactifs avant la réaction de fluoration, en utilisant des échangeurs de chaleur. On connait par CN101074185 la fluoration du 1,1,1,3-tetrachloropropane en trifluoropropène en présence de HF préchauffé à une température de 120°C à 230°C. On connait également par WO 2013/088195 la fluoration du 1,1,1,2,3-pentachloropropane en 2,3,3,3-tétrafluoropropène. On connait par WO 2014/159975 la préparation de 2-chloro-3,3,3-trifluoropropène à partir du 1,1,2,3-tétrachloropropène. On connait également par Thakur et al., Chemical Engineering Research and Design, vol. 81, no 7, p 787-826 que des mélangeurs statiques peuvent être utilisés pour mélanger des fluides.

Toutefois, cette étape préliminaire de chauffage, vaporisation et sur-chauffage des réactifs a tendance à conduire à la production de coke dans les échangeurs de chaleur.

Il existe donc un besoin de mettre au point un procédé de production de composés fluorés limitant ou évitant le problème du cokage de l'installation.

### RESUME DE L'INVENTION

L'invention concerne en premier lieu un procédé de production d'un composé fluoré comprenant :
- la fourniture d'un flux gazeux comprenant de l'acide fluorhydrique ;
- la fourniture d'au moins un flux liquide de composé chloré et la vaporisation de celui-ci par mélange avec ledit flux gazeux, le mélange résultant étant un mélange gazeux ;
- la fluoration catalytique du composé chloré avec l'acide fluorhydrique en phase gazeuse et la collecte d'un flux de produit ;
   caractérisé en ce que
- le mélange du flux liquide de composé chloré avec le flux gazeux comprenant de l'acide fluorhydrique est effectué dans un mélangeur statique ;
- le flux gazeux comprenant de l'acide fluorhydrique est à une température de 250 à 380°C au moment de son mélange avec le flux liquide de composé chloré ;
- le composé chloré est choisi parmi le 1,1,2-trichloroéthane, le 1,1,1,2,3-pentachloropropane, le 1,1,1,3,3-pentachloropropane, le 1,1,2,2,3-pentachloropropane, le 2,3-dichloro-1,1,1 -trifluoropropane, le perchloroéthylène, le 1,2-dichloroéthylène, le 1,1,2,3-tétrachloropropène, le 2,3,3,3-tetrachloropropène, le 1,1,3,3-tétrachloropropène, le 1,3,3,3-tétrachloropropène, le 2-chloro-3,3,3-trifluoropropène, le 1-chloro-3,3,3-trifluoropropène et leurs mélanges ; et
- le composé fluoré est choisi parmi le pentafluoroéthane, le 1-chloro,2,2-difluoroéthane, le 1,3,3,3-tétrafluoropropène, le 2,3,3,3-tétrafluoropropène, le 2-chloro-3,3,3-trifluoropropène, le 1-chloro-3,3,3-trifluoropropène et leurs mélanges.

Selon un mode de réalisation, le composé chloré est le perchloroéthylène et le composé fluoré est le pentafluoroéthane, ou le composé chloré est le 1,1,1,2,3-pentachloropropane et le composé fluoré est le 2,3,3,3-tétrafluoropropène.

Selon un autre mode de réalisation, le composé chloré est le 1,1,3,3-tétrachloropropène et le composé fluoré est le 1-chloro-3,3,3-trifluoropropène ; ou le composé chloré est le 1-chloro-3,3,3-trifluoropropène et le composé fluoré est le 1,3,3,3-tétrafluoropropène ; ou le composé chloré est le 1,1,2-trichloroéthane et le composé fluoré est le 1-chloro,2,2-difluoroéthane.

Selon l'invention, le mélange du flux liquide de composé chloré avec le flux gazeux comprenant de l'acide fluorhydrique est effectué dans un mélangeur statique.

Selon un mode de réalisation, le procédé comprend une ou des étapes de séparation du flux de produit, permettant de collecter d'une part un flux du composé fluoré et d'autre part un flux de recyclage.

Selon un mode de réalisation, le flux de recyclage fournit le flux gazeux comprenant de l'acide fluorhydrique, éventuellement après un apport d'acide fluorhydrique.

Selon un mode de réalisation, le procédé comprend une étape de fluoration catalytique du flux de recyclage, le cas échéant avec un apport d'acide fluorhydrique, le flux gazeux comprenant de l'acide fluorhydrique étant collecté à l'issue de cette étape de fluoration.

Selon un mode de réalisation, le procédé comprend une étape de chauffage du flux liquide de composé chloré à une température inférieure à la température de vaporisation de celui-ci.

Selon un mode de réalisation, le procédé comprend, après l'étape de mélange du flux liquide de composé chloré avec le flux gazeux comprenant de l'acide fluorhydrique, et avant l'étape de réaction catalytique du composé chloré avec l'acide fluorhydrique :
- une étape de chauffage du mélange ; ou
- une étape de refroidissement du mélange.

La présente divulgation concerne également une installation de production d'un composé fluoré comprenant :
- une conduite d'amenée de flux liquide de composé chloré ;
- une conduite d'amenée de flux gazeux comprenant de l'acide fluorhydrique ;
- une unité de mélange et de vaporisation alimentée par la conduite d'amenée de flux liquide de composé chloré et la conduite d'amenée de flux gazeux comprenant de l'acide fluorhydrique ;
- une conduite de collecte de mélange gazeux en sortie de l'unité de mélange et de vaporisation ;
- un réacteur de fluoration catalytique alimenté par la conduite de collecte de mélange gazeux ; et
- une conduite de collecte de flux de produit en sortie du réacteur de fluoration catalytique
caractérisé en ce que l'unité de mélange et de vaporisation (4) est un mélangeur statique.

Selon un mode de réalisation, l'installation comprend :
- au moins une unité de séparation alimentée par la conduite de collecte de flux de produits ; et
- une conduite de collecte de composé fluoré et une conduite de collecte de flux de recyclage en sortie de la ou les unités de séparation.

Selon un mode de réalisation, la conduite de collecte de flux de recyclage et éventuellement une conduite d'apport d'acide fluorhydrique alimentent la conduite d'amenée de flux gazeux comprenant de l'acide fluorhydrique.

Selon un mode de réalisation, l'installation comprend un réacteur de fluoration catalytique alimenté au moins en partie par la conduite de collecte de flux de recyclage, le cas échéant avec un apport d'acide fluorhydrique, la conduite d'amenée de flux gazeux comprenant de l'acide fluorhydrique étant issue du réacteur de fluoration catalytique.

Selon un mode de réalisation, l'installation comprend des moyens de chauffage sur la conduite d'amenée de flux liquide de composé chloré.

Selon un mode de réalisation, l'installation comprend des moyens de chauffage ou des moyens de refroidissement sur la conduite de collecte de mélange gazeux.

La présente invention permet de surmonter les inconvénients de l'état de la technique. Elle fournit plus particulièrement un procédé de production de composés fluorés limitant ou évitant le problème du cokage de l'installation.

Cela est accompli en vaporisant le principal réactif (composé chloré destiné à être fluoré) en le mélangeant avec un flux chaud gazeux qui contient de l'acide fluorhydrique.

On évite ainsi de vaporiser et surchauffer le principal réactif dans un échangeur de chaleur, qui présente une surface de contact très élevée ainsi que des points chauds, deux facteurs qui conduisent à un cokage important (le métal chaud ayant tendance à catalyser le cokage).

Par ailleurs, du fait de cette étape de mélange, la pression partielle du composé chloré lors de sa vaporisation est relativement modérée, et donc la température de vaporisation est également relativement modérée, et en tout cas inférieure à la température de vaporisation dans l'hypothèse où le composé chloré est vaporisé de manière indépendante. Cela permet notamment de limiter les risques de dégradation du composé chloré.

De manière générale, la température du flux gazeux comprenant de l'acide fluorhydrique, au moment de son mélange avec le flux liquide de composé chloré, est choisie :
- inférieure ou égale à la température de la réaction catalytique ;
- supérieure ou égale à la température de vaporisation du flux gazeux comprenant de l'acide fluorhydrique, qui est fonction de la pression et de la composition de ce flux (notamment teneur en HF).

Par exemple, dans le cadre de la production de HFC-125 (telle que décrite plus en détail ci-dessous), la température du flux gazeux comprenant de l'acide fluorhydrique peut être d'environ 165°C. Dans le cadre de la production de HFO-1234yf (telle que décrite plus en détail ci-dessous), la température du flux gazeux comprenant de l'acide fluorhydrique peut être d'environ 320 à 380°C. Selon l'invention, le flux gazeux comprenant de l'acide fluorhydrique est à une température de 250 à 380 °C au moment de son mélange avec le flux liquide de composé chloré.

### BREVE DESCRIPTION DES FIGURES

La **figure 1** représente de manière schématique un mode de réalisation de l'installation selon la présente divulgation.
La **figure 2** représente de manière schématique un autre mode de réalisation de l'installation selon la présente divulgation.

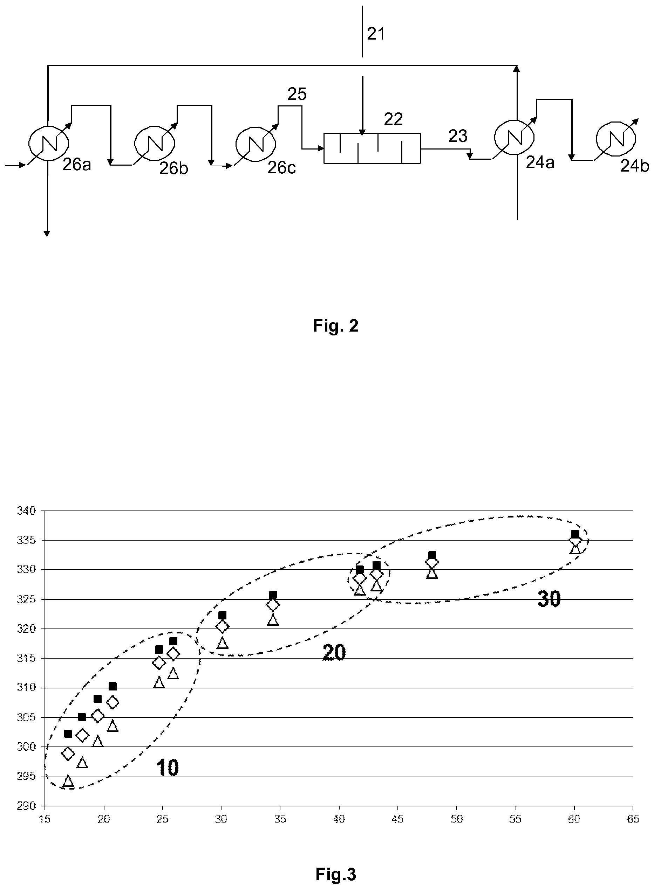

La **figure 3** est un graphe illustrant l'évolution de la température d'un flux d'alimentation du réacteur de fluoration principal après vaporisation du composé chloré (cf. exemple 1). En abscisse figure le débit du flux gazeux comprenant du HF, exprimé en t/h ; en ordonnée figure la température du flux après mélange avec le composé chloré et vaporisation de celui-ci, exprimée en °C. Les points représentés par Δ correspondent à une température initiale du composé chloré de 25°C, ceux représentés par ■ correspondent à une température initiale du composé chloré de 70°C et ceux représentés par ◇ correspondent à une température initiale du composé chloré de 100°C. Les trois groupes de données marqués 10, 20 et 30 sur le graphe correspondent à des rapports molaires HF/organiques valant respectivement 10, 20 et 30.

### DESCRIPTION DE MODES DE REALISATION DE LA PRESENTE DIVULGATION

La présente divulgation est maintenant décrite plus en détail et de façon non limitative dans la description qui suit.

La présente divulgation concerne la fluoration d'un composé chloré par de l'acide fluorhydrique, pour former un composé fluoré.

Par composé chloré, on entend un composé organique comprenant un ou plusieurs atomes de chlore, et par composé fluoré, on entend un composé organique comprenant un ou plusieurs atomes de fluor.

Il est entendu que le composé chloré peut comprendre un ou plusieurs atomes de fluor, et que le composé fluoré peut comprendre un ou plusieurs atomes de chlore. De manière générale, le nombre d'atomes de chlore du composé fluoré est inférieur au nombre d'atomes de chlore du composé chloré ; et le nombre d'atomes de fluor du composé fluoré est supérieur au nombre d'atomes de fluor du composé chloré.

Le composé chloré peut être un alcane ou un alcène ayant éventuellement des substituants choisis parmi F, Cl, I et Br (de préférence parmi F et Cl), et comportant au moins un substituant Cl.

Le composé fluoré peut être un alcane ou un alcène ayant éventuellement des substituants choisis parmi F, Cl, I et Br (de préférence parmi F et Cl), et comportant au moins un substituant F.

Le composé chloré peut notamment être un alcane avec un ou plusieurs substituants chlore (hydrochlorocarbure ou chlorocarbure) ou un alcane avec un ou plusieurs substituants chlore et fluor (hydrochlorofluorocarbure ou chlorofluorocarbure) ou un alcène avec un ou plusieurs substituants chlore (chlorooléfine ou hydrochlorooléfine) ou un alcène avec un ou plusieurs substituants chlore et fluor (hydrochlorofluorooléfine ou chlorofluorooléfine).

Le composé fluoré peut notamment être un alcane avec un ou plusieurs substituants fluor (fluorocarbure ou hydrofluorocarbure) ou un alcane avec un ou plusieurs substituants chlore et fluor (hydrochlorofluorocarbure ou chlorofluorocarbure) ou un alcène avec un ou plusieurs substituants fluor (fluorooléfine ou hydrofluorooléfine) ou un alcène avec un ou plusieurs substituants chlore et fluor (hydrochlorofluorooléfine ou chlorofluorooléfine).

Le composé chloré et le composé fluoré peuvent être linéaires ou ramifiés, de préférence linéaires.

Selon un mode de réalisation, le composé chloré et le composé fluoré comportent un seul atome de carbone.

Selon un mode de réalisation, le composé chloré et le composé fluoré comportent deux atomes de carbone.

Selon un mode de réalisation, le composé chloré et le composé fluoré comportent trois atomes de carbone.

Selon un mode de réalisation, le composé chloré et le composé fluoré comportent quatre atomes de carbone.

Selon un mode de réalisation, le composé chloré et le composé fluoré comportent cinq atomes de carbone.

La présente divulgation trouve notamment à s'appliquer pour les réactions de fluoration suivantes :
- fluoration du perchloroéthylène (PER) en pentafluoroéthane (HFC-125) ;
- fluoration du 1,1,1,2,3-pentachloropropane (HCC-240db) en 2-chloro-3,3,3-trifluoropropène (HCFO-1233xf) ;
- fluoration du 1,1,1,2,3-pentachloropropane (HCC-240db) en 2,3,3,3-tétrafluoropropène (HFO-1234yf) ;
- fluoration du 1,1,1,3,3-pentachloropropane (HCC-240fa) en 1,3,3,3-tétrafluoropropène (HFO-1234ze) ;
- fluoration du 1,1,1,3,3-pentachloropropane (HCC-240fa) en 1-chloro-3,3,3-trifluoropropène (HCFO-1233zd) ;
- fluoration du 2-chloro-3,3,3-trifluoropropène (HCFO-1233xf) en 2,3,3,3-tétrafluoropropène (HFO-1234yf);
- fluoration du 1,1,2,2,3-pentachloropropane (HCC-240aa) en 2,3,3,3-tétrafluoropropène (HFO-1234yf) ;
- fluoration du 1,1,2,2,3-pentachloropropane (HCC-240aa) en 2-chloro-3,3,3-trifluoropropène (HCFO-1233xf) ;
- fluoration du 2,3-dichloro-1,1,1-trifluoropropane (HCFC-243db) en 2,3,3,3-tétrafluoropropène (HFO-1234yf) ;
- fluoration du 2,3-dichloro-1,1,1-trifluoropropane (HCFC-243db) en 1-chloro-3,3,3-trifluoropropène (HCFO-1233zd) ;
- fluoration du 2,3-dichloro-1,1,1-trifluoropropane (HCFC-243db) en 1,3,3,3-tétrafluoropropène (HFO-1234ze) ;
- fluoration du 2,3-dichloro-1,1,1-trifluoropropane (HCFC-243db) en 2-chloro-3,3,3-trifluoropropène (HCFO-1233xf) ;
- fluoration du 1,1,2,3-tétrachloropropène (HCO-1230xa) en 2,3,3,3-tétrafluoropropène (HFO-1234yf) ;
- fluoration du 1,1,2,3-tétrachloropropène (HCO-1230xa) en 2-chloro-3,3,3-trifluoropropène (HCFO-1233xf) ;
- fluoration du 2,3,3,3-tetrachloropropène (HCO-1230xf) en 2,3,3,3-tétrafluoropropène (HFO-1234yf) ;
- fluoration du 2,3,3,3-tétrachloropropène (HCO-1230xf) en 2-chloro-3,3,3-trifluoropropène (HCFO-1233xf) ;
- fluoration du 1,1,3,3-tétrachloropropène (HCO-1230za) en 1-chloro-3,3,3-trifluoropropène (HCFO-1233zd) ;
- fluoration du 1,1,3,3-tétrachloropropène (HCO-1230za) en 1,3,3,3-tétrafluoropropène (HFO-1234ze) ;
- fluoration du 1,3,3,3-tétrachloropropène (HCO-1230zd) en 1-chloro-3,3,3-trifluoropropène (HCFO-1233zd) ;
- fluoration du 1,3,3,3-tétrachloropropène (HCO-1230zd) en 1,3,3,3-tétrafluoropropène (HFO-1234ze) ;
- fluoration du 1-chloro-3,3,3-trifluoropropène (HCFO-1233zd) en 1,3,3,3-tétrafluoropropène (HFO-1234ze) ;
- fluoration du 1,1,2-trichloroéthane en 1-chloro,2,2-difluoroéthane (HCFC-142) ;
- fluoration du 1,2-dichloroéthylène en 1-chloro-2,2-difluoroéthane (HCFC-142)

La conversion du composé chloré en composé fluoré peut être une conversion directe (avec une seule étape de réaction ou avec un seul ensemble de conditions réactionnelles) ou une conversion indirecte (avec deux ou plus de deux étapes de réaction ou en utilisant deux ou plus de deux ensembles de conditions réactionnelles).

La réaction de fluoration peut être effectuée :
- avec un rapport molaire HF / composé chloré de 3:1 à 150:1, de préférence de 4:1 à 100:1 et de manière plus particulièrement préférée de 5:1 à 50:1 ;
- avec un temps de contact de 1 à 100 s, de préférence 1 à 50 s et plus particulièrement 2 à 40 s (volume de catalyseur divisé par le flux entrant total, ajusté à la température et à la pression de fonctionnement) ;
- à une pression absolue allant de 0,1 à 50 bar, de préférence de 0,3 à 15 bar ;
- à une température (température du lit de catalyseur) de 100 à 500°C, de préférence de de 200 à 450°C, et plus particulièrement de 300 à 400°C.

Afin d'éviter une désactivation rapide du catalyseur lors de la réaction, un agent d'oxydation (par exemple de l'oxygène ou du chlore) peut être ajouté, par exemple dans un rapport molaire agent d'oxydation / composés organiques de 0,005 à 2, de préférence de 0,01 à 1,5. On peut par exemple utiliser un flux d'oxygène pur ou de chlore pur, ou un mélange oxygène / azote ou chlore / azote.

Le catalyseur utilisé peut être par exemple à base d'un métal comprenant un oxyde de métal de transition ou un dérivé ou un halogénure ou un oxyhalogénure d'un tel métal. On peut citer par exemple FeCl₃, l'oxyfluorure de chrome, les oxydes de chrome (éventuellement soumis à des traitements de fluoration), les fluorures de chrome et leurs mélanges. D'autres catalyseurs possibles sont les catalyseurs supportés sur du carbone, les catalyseurs à base d'antimoine, les catalyseurs à base d'aluminium (par exemple AlF₃ et Al₂O₃, l'oxyfluorure d'alumine et le fluorure d'alumine).

On peut utiliser en général un oxyfluorure de chrome, un fluorure ou un oxyfluorure d'aluminium, ou un catalyseur supporté ou non contenant un métal tel que Cr, Ni, Fe, Zn, Ti, V, Zr, Mo, Ge, Sn, Pb, Mg, Sb.

On peut faire référence à cet égard au document WO 2007/079431 (en p.7, I.1-5 et 28-32), au document EP 939071 (paragraphe [0022]), au document WO 2008/054781 (en p.9 I.22-p.10 I.34), et au document WO 2008/040969 (revendication 1), auxquels il est fait expressément référence.

Avant son utilisation, le catalyseur est de préférence soumis à une activation avec de l'air, de l'oxygène ou du chlore et/ou avec de l'HF.

Avant son utilisation, le catalyseur est de préférence soumis à une activation avec de l'air ou de l'oxygène et du HF à une température de 100 à 500°C, de préférence de 250 à 500°C et plus particulièrement de 300 à 400°C. La durée d'activation est de préférence de 1 à 200 h et plus particulièrement de 1 à 50 h.

Cette activation peut être suivie d'une étape d'activation de fluoration finale en présence d'un agent d'oxydation, d'HF et de composés organiques.

Le rapport molaire HF / composés organiques est de préférence de 2 à 40 et le rapport molaire agent d'oxydation / composés organiques est de préférence de 0,04 à 25. La température de l'activation finale est de préférence de 300 to 400°C and sa durée de préférence de 6 à 100 h.

Le catalyseur est de préférence à base de chrome et il s'agit plus particulièrement d'un catalyseur mixte comprenant du chrome.

Selon un mode de réalisation, on utilise un catalyseur mixte comprenant du chrome et du nickel. Le rapport molaire Cr / Ni (sur la base de l'élément métallique) est généralement de 0,5 à 5, par exemple de 0,7 à 2, par exemple d'environ 1. Le catalyseur peut contenir de 0,5 à 20 % en poids de chrome, et de 0,5 à 20 % en poids de nickel, de préférence de 2 à 10 % de chaque.

Le métal peut être présent sous forme métallique ou sous forme de dérivé, par exemple un oxyde, halogénure ou oxyhalogénure. Ces dérivés sont de préférence obtenus par activation du métal catalytique.

Le support est de préférence constitué avec de l'aluminium, par exemple de l'alumine, de l'alumine activée ou des dérivés d'aluminium, tels que les halogénures d'aluminium et les oxyhalogénures d'aluminium, par exemple décrits dans le document US 4,902,838, ou obtenus par le procédé d'activation décrit ci-dessus.

Le catalyseur peut comprendre du chrome et du nickel sous une forme activée ou non, sur un support qui a été soumis à une activation ou non.

On peut se reporter au document WO 2009/118628 (notamment en p.4, I.30-p.7 I.16), auquel il est fait expressément référence ici.

Un autre mode de réalisation préféré repose sur un catalyseur mixte contenant du chrome et au moins un élément choisi parmi Mg et Zn. Le rapport atomique de Mg ou Zn/Cr est de préférence de 0,01 à 5.

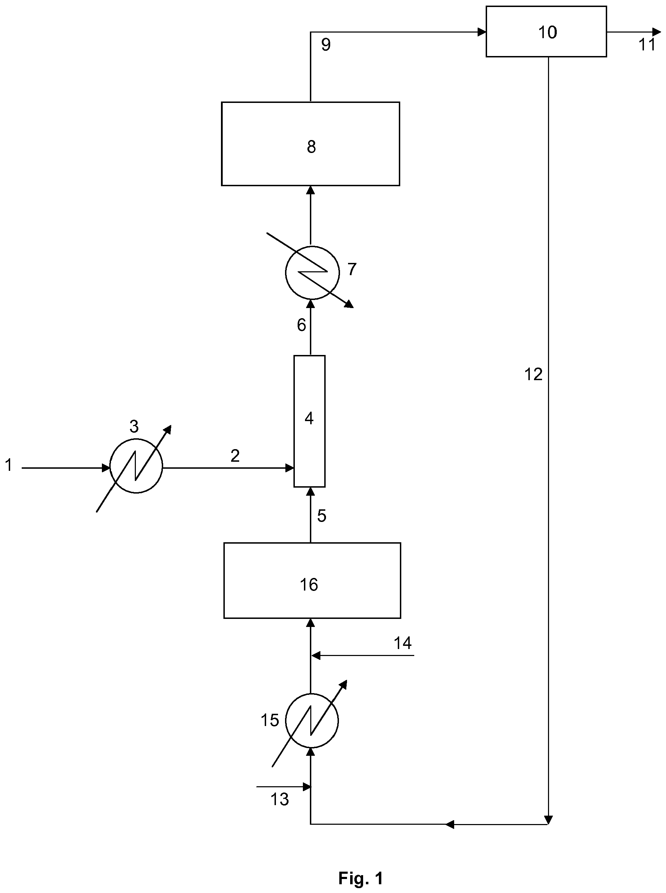

En faisant référence à la **figure 1**, un mode de réalisation de l'invention est maintenant décrit dans le cas particulier d'un procédé de production de HFO-1234yf à partir de HCC-240db, étant entendu qu'il vaut de manière analogue avec d'autres couples de composés chlorés / composés fluorés.

L'installation selon la présente divulgation comprend une conduite d'amenée de flux liquide de HCC-240db 2 et une conduite d'amenée de flux gazeux comprenant de l'HF 5, qui alimentent un réacteur de fluoration catalytique 8. La conduite d'amenée de flux liquide de HCC-240db 2 est issue d'une réserve de HCC-240db liquide 1. La conduite d'amenée de flux gazeux comprenant de l'HF 5 peut transporter un flux de HF pur (éventuellement en combinaison avec un agent d'oxydation tel que décrit ci-dessus) ou, alternativement, un mélange de HF et de composés organiques, notamment de composés organiques chloré et/ou fluorés, comme c'est le cas dans l'exemple illustré, et comme cela sera décrit plus en détail ci-dessous.

Une unité de mélange et de vaporisation 4 est alimentée à la fois par la conduite d'amenée de flux liquide de HCC-240db 2 et la conduite d'amenée de flux gazeux comprenant de l'HF 5. Cette unité est adaptée à mélanger le flux gazeux et le flux liquide. Il s'agit de préférence d'un mélangeur statique afin de permettre un procédé de type continu. Dans cette unité, le flux gazeux comprenant de l'HF cède de la chaleur au flux liquide de HCC-240db, ce qui permet la vaporisation du HCC-240db.

Le mélange du HCC-240db, du HF et éventuellement de composés supplémentaires est collecté dans une conduite de collecte de mélange gazeux 6 en sortie de l'unité de mélange et de vaporisation 4, qui transporte le mélange jusqu'au réacteur de fluoration catalytique 8.

Le HCC-240db peut subir une étape de chauffage préliminaire avant le mélange avec le flux gazeux comprenant de l'HF. Dans ce cas, ce chauffage préliminaire est effectué à une température inférieure à la température de vaporisation du HCC-240db (et à une température inférieure à la température de dégradation ou de décomposition de ce composé). A cet effet, on peut prévoir des moyens de chauffage 3 sur la conduite d'amenée de flux liquide de HCC-240db 2.

Entre le mélange du HCC-240db avec le flux comprenant de l'HF et la réaction de fluoration, on peut, selon les cas, prévoir un chauffage complémentaire du mélange ou au contraire un refroidissement de celui-ci, en prévoyant soit des moyens de chauffage soit, comme illustré sur la figure, des moyens de refroidissement 7 sur la conduite de collecte de mélange gazeux 6. Le choix du chauffage ou du refroidissement dépend de la température souhaitée pour la réaction de fluoration, en comparaison de la température du mélange gazeux issu de l'unité de mélange et de vaporisation 4.

En sortie du réacteur de fluoration catalytique 8 est connectée une conduite de collecte de flux de produits 9. Celle-ci alimente une unité de séparation 10 (ou plusieurs unités de séparation successives) permettant notamment de séparer le produit d'intérêt (composé fluoré, ici le HFO-1234yf) du reste du flux de produits. A cet égard, on peut notamment utiliser une ou plusieurs colonnes de distillation, ou des unités de décantation, extraction, lavage ou autres. Ce produit d'intérêt est récupéré dans une conduite de collecte de composé fluoré 11 en sortie de l'unité de séparation 10. Par ailleurs, un flux de recyclage est récupéré dans une conduite de collecte de flux de recyclage 12. D'autres produits indésirables peuvent être éliminés par ailleurs à ce stade (notamment l'acide chlorhydrique généré lors de la réaction de fluoration).

Le flux de recyclage peut contenir notamment des réactifs non réagis, à savoir HF et composé chloré (ici le HCC-240db). Il peut également contenir des produits secondaires issus de la réaction, c'est-à-dire des produits fluorés obtenus par fluoration du composé chloré (HCC-240db) et autres que le composé fluoré désiré. Dans le cas illustré, le flux de recyclage contient notamment du HCFO-1233xf, et éventuellement du HFC-245cb (1,1,1,2,2-pentafluoropropane), obtenus par fluoration du HCC-240db.

Selon un mode de réalisation possible, le flux de recyclage peut être directement retourné vers le réacteur de fluoration catalytique 8. Selon un autre mode de réalisation possible, il peut faire l'objet d'un traitement complètement distinct, voire d'une valorisation séparée. Selon un autre mode de réalisation possible, il est partiellement retourné au réacteur de fluoration catalytique 8.

Selon un autre de mode réalisation, qui est celui illustré ici, le flux de recyclage subit une fluoration complémentaire avant d'être retourné vers le réacteur de fluoration catalytique 8 principal.

Ainsi, la conduite de collecte de flux de recyclage 12 alimente un réacteur de fluoration catalytique 16 complémentaire. Une conduite d'apport d'HF 13 peut le cas échéant, comme illustré, être connectée sur celle-ci afin d'apporter de l'HF frais. Une conduite d'apport d'agent oxydant 14 peut aussi, le cas échéant, comme illustré, être connectée sur conduite de collecte de flux de recyclage 12 afin d'assurer un apport en agent oxydant propre à maintenir l'activité catalytique du catalyseur.

Des moyens de chauffage et de vaporisation 15 peuvent être prévus sur la conduite de collecte de flux de recyclage 12 afin de porter le flux à la température souhaitée pour la réaction de fluoration complémentaire, qui est effectuée dans le réacteur de fluoration catalytique 16 complémentaire.

Dans l'exemple illustré, la conduite d'amenée de flux gazeux comprenant de l'HF 5 (précédemment décrite) est directement issue du réacteur de fluoration catalytique 16 complémentaire. Ainsi, le flux gazeux comprenant de l'HF contient, outre de l'HF (et le cas échéant de l'agent oxydant), des produits fluorés issus de la réaction de fluoration complémentaire.

Un apport de HF frais et/ou un apport d'agent oxydant peuvent être ajoutés dans la conduite d'amenée de flux gazeux comprenant de l'HF 5 si besoin.

Le principe d'un procédé de production comprenant deux étapes distinctes de fluoration catalytique, l'alimentation en réactif chloré (HCC-240db) s'effectuant entre ces deux étapes, est décrit en détail dans le document WO 2013/088195, auquel il est renvoyé à titre de référence.

Dans la description qui précède, le flux gazeux comprenant du HF (qui est utilisé pour vaporiser le flux liquide de composé chloré) correspond à un flux issu d'une réaction de fluoration complémentaire d'un flux de recyclage. D'autres variantes sont possibles :
- le flux gazeux comprenant du HF peut être un flux issu d'une réaction de fluoration complémentaire d'un flux de recyclage, additionné de HF supplémentaire et/ou d'agent d'oxydation supplémentaire ;
- le flux gazeux comprenant du HF peut être directement un flux de recyclage ou un flux partiel de recyclage (sans étape de réaction de fluoration complémentaire);
- le flux gazeux comprenant du HF peut être directement un flux de recyclage (sans étape de réaction de fluoration complémentaire), additionné de HF supplémentaire et/ou d'agent d'oxydation supplémentaire ;
- le flux gazeux comprenant du HF peut être un flux de HF frais comprenant éventuellement de l'agent d'oxydation frais.

Dans ce dernier cas, si un flux de recyclage est présent, il peut être introduit après l'étape de mélange du flux gazeux comprenant du HF avec le flux liquide de composé chloré ; et, si on met en œuvre une réaction de fluoration complémentaire d'un flux de recyclage, le flux issu de cette réaction peut être introduit après l'étape de mélange du flux gazeux comprenant du HF avec le flux liquide de composé chloré.

Un autre mode de réalisation est maintenant décrit en faisant référence à la **figure 2** : il s'agit d'un procédé de production de HFC-125 à partir de PER (ainsi que l'installation permettant sa mise en œuvre).

L'installation comporte une conduite d'amenée de flux gazeux comprenant du HF 25 et une conduite d'amenée de flux liquide de PER 21, qui alimentent toutes deux une unité de mélange et de vaporisation 22, qui est un mélangeur statique. En sortie de celui-ci est connectée une conduite de collecte de mélange gazeux 23, qui alimente un ou une série de plusieurs réacteurs de fluoration (non représentés).

Des moyens de chauffage 26a, 26b, 26c sont prévus sur la conduite d'amenée de flux gazeux comprenant du HF 25. Des moyens de chauffage 24a, 24b sont prévus sur la conduite de collecte de mélange gazeux 23.

Selon un mode de réalisation, le flux gazeux comprenant du HF est obtenu par chauffage et le cas échéant vaporisation d'un flux de recyclage collecté après traitement et séparation d'un flux de produits issu de la réaction de fluoration catalytique.

Certains des moyens de chauffage 26a, 24a employés peuvent être des échangeurs économiseurs de chaleur.

Les paramètres importants à prendre en compte de manière générale dans la mise en œuvre du procédé de la présente divulgation sont les suivants :
- le débit de flux gazeux comprenant du HF doit être supérieur au débit de composé chloré, et suffisamment grand par rapport à celui-ci afin de permettre une vaporisation complète du composé chloré et éviter une condensation partielle du flux gazeux comprenant du HF ; ainsi, de préférence, le rapport de débits massiques entre le flux gazeux comprenant du HF et le flux de composé chloré est de 1 à 30, de préférence de 1,2 à 25 et de manière plus particulièrement préférée de 1,5 à 20 ;
- la température du flux gazeux comprenant du HF doit être suffisamment élevée, pour les mêmes raisons (elle doit en tout état de cause être supérieure à la température de vaporisation du composé chloré, à la pression considérée) ;
- le différentiel entre la température du flux gazeux comprenant du HF et la température le mélange gazeux après vaporisation du composé chloré doit rester relativement faible, de préférence inférieur ou égal à 50°C, ou inférieure ou égal à 30°C ou à 25°C.

Lorsque la température du flux gazeux comprenant du HF est relativement faible (de l'ordre de 150 ou 200°C par exemple), un rapport massique de débit (flux gazeux / flux liquide) relativement élevé est nécessaire pour assurer une vaporisation complète du composé chloré. Mais le différentiel de température résultant est relativement faible. Dans ce régime, la chaleur générée par l'oligomérisation du HF est utilisée pour vaporiser le composé chloré.

Lorsque la température du flux gazeux comprenant du HF est relativement élevée (de l'ordre de 250 ou 300°C par exemple), un rapport massique de débit plus faible est nécessaire, mais le différentiel de température obtenu est relativement élevé. Dans ce régime, la vapeur de HF n'est pas sous forme oligomère, et la chaleur de vaporisation du composé chloré est fournie par le refroidissement du HF surchauffé.

### EXEMPLES

Les exemples suivants illustrent l'invention sans la limiter.

### Exemple 1 - étude de l'abaissement de température du flux de HF lié au mélange avec le composé chloré

Pour cette étude, on considère une réaction de fluoration de HCC-240db en HFO-1234yf selon le schéma de la **figure 1****.**

On considère une productivité de HFO-1234yf de 1600 kg/h, une réaction de fluoration (réacteur 8) effectuée à 4, 5 ou 7 bar absolus selon les cas, un rapport molaire HF/composé chloré de 10, 20 ou 30 selon les cas, et un taux de conversion du HCFO-1233xf de 60 ou 70 % selon les cas. Le débit d'alimentation en HCC-240db est de 3100 kg/h dans tous les cas.

On considère que la réaction de fluoration préliminaire (réacteur 16) est effectuée à une température de 350°C, et donc que le flux gazeux comprenant du HF (conduite 5) est à cette température de 350°C.

On considère trois hypothèses concernant la température du flux de HCC-240db : 25°C (absence de préchauffage du flux), 70°C ou 100°C.

A partir de l'ensemble de ces hypothèses, on calcule dans chaque cas la température du flux après mélange entre le flux comprenant du HF et le flux liquide de HCC-240db, à partir des données disponibles sur la pression de vapeur en fonction de la température pour le HCC-240db. Les résultats sont représentés sur la **figure 3****.**

On constate que l'influence du préchauffage du flux de HCC-240db sur la température finale est relativement faible (différence de 2,4 à 8°C sur la température finale du flux après mélange, selon que le flux de HCC-240db est préchauffé ou non). La nécessité de prévoir un chauffage complémentaire (ou au contraire un refroidissement) du mélange avant la réaction dépend de la température de réaction souhaitée.

On constate, de manière générale, que la diminution de température liée au mélange avec le HCC-240db et à la vaporisation de celui-ci est modérée et compatible avec la mise en œuvre du procédé.

### Exemple 2 - étude du rapport massique de débit nécessaire entre le flux gazeux comprenant du HF et le flux de composé chloré

Pour cette étude, on utilise les mêmes hypothèses de base que dans l'exemple 1, en prenant une pression de 7 bar absolus. On envisage ici des flux de HF (pur) à différentes températures, à savoir 150, 200, 250 ou 300°C. Selon les cas, on admet une diminution plus ou moins forte de la température suite au mélange avec le HCC-240db (notée ΔT, en valeur absolue), et on en déduit le rapport massique de débit (R) correspondant (débit massique de flux gazeux comprenant du HF sur débit massique de HCC-240db).

On calcule également les valeurs limites de ΔT et R permettant la vaporisation complète du HCC-240db (sans entraîner de condensation du flux gazeux comprenant du HF). Les résultats sont résumés dans le tableau ci-dessous :

| | ΔT=50°C | ΔT=30°C | ΔT=25°C | Limite pour vaporisation complète |
|---|---|---|---|---|
| Flux HF à 150°C | Impossible | Impossible | R=2,9 | R=2,6 (soit ΔT=26°C) |
| Flux HF à 200°C | R=2,9 | R=5,6 | R=6,9 | R=1,8 (soit ΔT=66°C) |
| Flux HF à 250°C | R=4,3 | R=7,6 | R=9,4 | R=1,3 (soit ΔT=109°C) |
| Flux HF à 300°C | R=5,1 | R=9,0 | R=10,9 | R=1,1 (soit ΔT=152°C) |

### Exemple 3 - Essai pilote

Un flux gazeux issu d'un réacteur de fluoration comprenant de l'HF est mélangé à un flux liquide de 240db préalablement préchauffé. Le débit du flux gazeux comprenant de l'HF issu du réacteur de fluoration est de 20 à 50 kg/h. Ce flux gazeux est à une température de 320 °C à 350°C et à une pression 3 à 5 de bara. Le débit du flux liquide de 240db est de 3 à 4 kg/h. Ce flux liquide de 240db est préchauffé à une température de 100°C à 120°C à une pression de 4 à 6 bara.

Lorsque les deux flux sont mélangés, le flux liquide de 240db est instantanément vaporisé par le flux comprenant de l'HF et la température du flux gazeux résultant du mélange de ces deux flux est de 280°C à 330 °C à 3 à 5 bara. Ce flux résultant peut éventuellement être réchauffé à une température de 350°C à 380°C avant d'alimenter un autre réacteur de fluoration dans lequel est opéré la fluoration du 240db en 1233xf.

## Revendications

1. Procédé de production d'un composé fluoré comprenant :
- la fourniture d'un flux gazeux comprenant de l'acide fluorhydrique ;
- la fourniture d'au moins un flux liquide de composé chloré et la vaporisation de celui-ci par mélange avec ledit flux gazeux, le mélange résultant étant un mélange gazeux ;
- la fluoration catalytique du composé chloré avec l'acide fluorhydrique en phase gazeuse pour former ledit composé fluoré et la collecte d'un flux de produit ;
**caractérisé en ce que**
le mélange du flux liquide de composé chloré avec le flux gazeux comprenant de l'acide fluorhydrique est effectué dans un mélangeur statique ;
le flux gazeux comprenant de l'acide fluorhydrique est à une température de 250 à 380°C au moment de son mélange avec le flux liquide de composé chloré ;
le composé chloré est choisi parmi le 1,1,2-trichloroéthane, le 1,1,1,2,3-pentachloropropane, le 1,1,1,3,3-pentachloropropane, le 1,1,2,2,3-pentachloropropane, le 2,3-dichloro-1,1,1-trifluoropropane, le perchloroéthylène, le 1,2-dichloroéthylène, le 1,1,2,3-tétrachloropropène, le 2,3,3,3-tetrachloropropène, le 1,1,3,3-tétrachloropropène, le 1,3,3,3-tétrachloropropène, le 2-chloro-3,3,3-trifluoropropène, le 1-chloro-3,3,3-trifluoropropène et leurs mélanges ; et
le composé fluoré est choisi parmi le pentafluoroéthane, le 1-chloro,2,2-difluoroéthane, le 1,3,3,3-tétrafluoropropène, le 2,3,3,3-tétrafluoropropène, le 2-chloro-3,3,3-trifluoropropène, le 1-chloro-3,3,3-trifluoropropène et leurs mélanges.

2. Procédé selon la revendication 1, dans lequel le composé chloré est le perchloroéthylène et le composé fluoré est le pentafluoroéthane, ou le composé chloré est le 1,1,1,2,3-pentachloropropane et le composé fluoré est le 2,3,3,3-tétrafluoropropène.

3. Procédé selon l'une des revendications 1 à 2, comprenant une ou des étapes de séparation du flux de produit, permettant de collecter d'une part un flux du composé fluoré et d'autre part un flux de recyclage.

4. Procédé selon la revendication 3, dans lequel le flux de recyclage fournit le flux gazeux comprenant de l'acide fluorhydrique, éventuellement après un apport d'acide fluorhydrique.

5. Procédé selon la revendication 3, comprenant une étape de fluoration catalytique du flux de recyclage, le cas échéant avec un apport d'acide fluorhydrique, le flux gazeux comprenant de l'acide fluorhydrique étant collecté à l'issue de cette étape de fluoration.

6. Procédé selon l'une des revendications 1 à 5, comprenant une étape de chauffage du flux liquide de composé chloré à une température inférieure à la température de vaporisation de celui-ci.

7. Procédé selon l'une des revendications 1 à 6, comprenant, après l'étape de mélange du flux liquide de composé chloré avec le flux gazeux comprenant de l'acide fluorhydrique, et avant l'étape de réaction catalytique du composé chloré avec l'acide fluorhydrique :
- une étape de chauffage du mélange ; ou
- une étape de refroidissement du mélange.

## Patentansprüche

1. Verfahren zur Herstellung einer Fluorverbindung, umfassend:
- das Bereitstellen eines gasförmigen Stroms, der Fluorwasserstoffsäure umfasst;
- das Bereitstellen mindestens eines flüssigen Stroms einer Chlorverbindung und das Verdampfen davon durch Mischen mit dem gasförmigen Strom, wobei es sich bei der resultierenden Mischung um eine gasförmige Mischung handelt;
- das katalytische Fluorieren der Chlorverbindung mit der Fluorwasserstoffsäure in der Gasphase zur Bildung der Fluorverbindung und das Sammeln eines Produktstroms;
**dadurch gekennzeichnet, dass**:
das Mischen des flüssigen Stroms einer Chlorverbindung mit dem gasförmigen Strom, der Fluorwasserstoffsäure umfasst, in einem statischen Mischer durchgeführt wird;
der Gasstrom, der Fluorwasserstoffsäure umfasst, zum Zeitpunkt des Mischens mit dem flüssigen Strom einer Chlorverbindung bei einer Temperatur von 250 bis 380 °C vorliegt;
die Chlorverbindung aus 1,1,2-Trichlorethan, 1,1,1,2,3-Pentachlorpropan, 1,1,1,3,3-Pentachlorpropan, 1,1,2,2,3-Pentachlorpropan, 2,3-Dichlor-1,1,1-trifluorpropan, Perchlorethylen, 1,2-Dichlorethylen, 1,1,2,3-Tetrachlorpropen, 2, 3, 3, 3-Tetrachlorpropen, 1,1,3,3-Tetrachlorpropen, 1,3,3,3-Tetrachlorpropen, 2-Chlor-3,3,3-trifluorpropen, 1-Chlor-3,3,3-trifluorpropen und Mischungen davon ausgewählt wird und
die Fluorverbindung aus Pentafluorethan, 1-Chlor-2,2-difluorethan, 1,3,3,3-Tetrafluorpropen, 2,3,3,3-Tetrafluorpropen, 2-Chlor-3,3,3-trifluorpropen, 1-Chlor-3,3,3-trifluorpropen und Mischungen davon ausgewählt wird.

2. Verfahren nach Anspruch 1, wobei es sich bei der Chlorverbindung um Perchlorethylen handelt und es sich bei der Fluorverbindung um Pentafluorethan handelt oder es sich bei der Chlorverbindung um 1,1,1,2,3-Pentachlorpropan handelt und es sich bei der Fluorverbindung um 2,3,3,3-Tetrafluorpropen handelt.

3. Verfahren nach einem der Ansprüche 1 bis 2, umfassend einen oder mehrere Schritte der Trennung des Produktstroms, wodurch einerseits ein Strom der Fluorverbindung und andererseits ein Rückführungsstrom gesammelt werden kann.

4. Verfahren nach Anspruch 3, wobei der Rückführungsstrom den gasförmigen Strom, der Fluorwasserstoffsäure umfasst, bereitstellt, gegebenenfalls nach Zusatz von Fluorwasserstoffsäure.

5. Verfahren nach Anspruch 3, umfassend einen Schritt der katalytischen Fluorierung des Rückführungsstroms gegebenenfalls unter Zusatz von Fluorwasserstoffsäure, wobei der gasförmige Strom, der Fluorwasserstoffsäure umfasst, am Ende dieses Fluorierungsschritts gesammelt wird.

6. Verfahren nach einem der Ansprüche 1 bis 5, umfassend einen Schritt des Erhitzens des flüssigen Stroms einer Chlorverbindung auf eine Temperatur unterhalb ihrer Verdampfungstemperatur.

7. Verfahren nach einem der Ansprüche 1 bis 6, umfassend, nach dem Schritt des Mischens des flüssigen Stroms einer Chlorverbindung mit dem gasförmigen Strom, der Fluorwasserstoffsäure umfasst, und vor dem Schritt der katalytischen Reaktion der Chlorverbindung mit der Fluorwasserstoffsäure,
- einen Schritt des Erhitzens der Mischung oder
- einen Schritt des Abkühlens der Mischung.

## Claims

1. Process for producing a fluoro compound, involving:
- supplying a gas stream comprising hydrofluoric acid;
- supplying at least one liquid stream of chloro compound and vaporizing said compound by mixing with said gas stream, the resulting mixture being a gaseous mixture;
- catalytically fluorinating the chloro compound with the hydrofluoric acid in the gas phase to form said fluoro compound and collecting a product stream; **characterized in that**
the mixing of the liquid stream of chloro compound with the gas stream comprising hydrofluoric acid is performed in a static mixer;
the gas stream comprising hydrofluoric acid is at a temperature from 250 to 380°C at the time of its mixing with the liquid stream of chloro compound; the chloro compound is chosen from 1,1,2-trichloroethane, 1,1,1,2,3-pentachloropropane, 1,1,1,3,3-pentachloropropane, 1,1,2,2,3-pentachloropropane, 2,3-dichloro-1,1,1-trifluoropropane, perchloroethylene, 1,2-dichloroethylene, 1,1,2,3-tetrachloropropene, 2,3,3,3-tetrachloropropene, 1,1,3,3-tetrachloropropene, 1,3,3,3-tetrachloropropene, 2-chloro-3,3,3-trifluoropropene and 1-chloro-3,3,3-trifluoropropene, and mixtures thereof; and
the fluoro compound is chosen from pentafluoroethane, 1-chloro-2,2-difluoroethane, 1, 3, 3, 3-tetrafluoropropene, 2,3,3,3-tetrafluoropropene, 2-chloro-3,3,3-trifluoropropene and 1-chloro-3,3,3-trifluoropropene, and mixtures thereof.

2. Process according to Claim 1, in which the chloro compound is perchloroethylene and the fluoro compound is pentafluoroethane, or the chloro compound is 1,1,1,2,3-pentachloropropane and the fluoro compound is 2,3,3,3-tetrafluoropropene.

3. Process according to either of Claims 1 and 2, comprising one or more steps for separation of the product stream, making it possible to collect, on the one hand, a stream of the fluoro compound and, on the other hand, a recycling stream.

4. Process according to Claim 3, in which the recycling stream provides the gas stream comprising hydrofluoric acid, optionally after supplying hydrofluoric acid.

5. Process according to Claim 3, comprising a step of catalytic fluorination of the recycling stream, where appropriate with a supply of hydrofluoric acid, the gas stream comprising hydrofluoric acid being collected on conclusion of this fluorination step.

6. Process according to one of Claims 1 to 5, comprising a step of heating the liquid stream of chloro compound to a temperature below its vaporization temperature.

7. Process according to one of Claims 1 to 6, comprising, after the step of mixing the liquid stream of chloro compound with the gas stream comprising hydrofluoric acid, and before the step of catalytic reaction of the chloro compound with the hydrofluoric acid:
- a step of heating the mixture; or
- a step of cooling the mixture.
